# EUROPEAN PATENT SPECIFICATION

(11) **EP 3 250 286 B1**
(45) Date of publication and mention of the grant of the patent: **14.12.2022**
(21) Application number: 16744231.8
(22) Date of filing: 29.01.2016
(51) Int. Cl.: A61N 1/36, A61N 1/04, A61N 1/05, A61N 1/378

(54) **NEUROSTIMULATOR SYSTEM**
NEUROSTIMULATORSYSTEM
SYSTÈME NEUROSTIMULATEUR

(30) Priority: 30.01.2015 US 201514610926
(43) Date of publication of application: 06.12.2017
(73) Proprietor: AdvaStim, Inc., Beverly, MA 01915 (US)
(72) Inventor: YOMTOV, Barry, M., Marblehead, MA 01945 (US)
(74) Representative: Pezzoli, Ennio
(86) International application number: PCT/US2016/015805
(87) International publication number: WO 2016/123563

(56) References cited:
- US-A1- 2010 069 992
- US-A1- 2010 114 278
- US-A1- 2011 034 964
- US-A1- 2011 137 378
- US-A1- 2012 215 287
- US-A1- 2013 238 048
- US-A1- 2013 245 733
- US-A1- 2014 180 370
- US-B2- 8 663 202

## Description

### BACKGROUND

An embodiment of the present invention generally relates to a lead for use in an implantable therapy system. The present invention more particularly relates to a stimulator lead having an integrated switching circuit and optionally also having an integrated pulse generator for efficiently providing an array of electrodes with stimulation energy from an implantable pulse generator.

Implantable therapy delivery systems have been in the art and in commercial use for decades. Such systems include cardiac rhythm management systems such pacemakers and defibrillators, nerve stimulators, and even drug delivery systems.

Such therapy systems, and especially in the case of cardiac rhythm management and nerve stimulator systems, include an implantable device that includes a power source, such as a battery and electronic circuitry that generates therapy stimulation pulses and controls when the therapy stimulation pulses are delivered. To actually deliver the stimulation pulses, the systems also generally include multiple stimulation electrodes on the surface of a lead that make electrical contact with the desired (target) tissue and a lead system, including one or more leads that connect the electrodes to the electronic circuitry in the device.

As implantable therapy device design has progressed over time, more and more functionality has been incorporated into the implantable devices and more and more electrodes have been similarly required to shape stimulation at the target tissue volume to enable that functionality. For example, implantable therapy devices usually now incorporate microcontrollers that are capable of controlling multiple therapy delivery modalities in multiple locations of the body. Those modalities may include both stimulation pulse delivery to selected tissue(s) and/or physiologic activity monitoring and data gathering for analysis and adjustment of therapy. In the case of nerve stimulation systems, these systems now find use in various locations of the body as for example, in brain tissue stimulation and spinal nerve stimulation.

Particularly in nerve stimulators, there has been an increase in the number of electrodes assigned to shape and deliver electrical pulses to a given anatomical region. The intended advantage is to obtain stimulation selectivity and directionality and to shape current delivery to a volume of tissue. Today, a system may incorporate as many as sixteen to twenty electrodes in a given area. Unfortunately, current state of the art connectivity measures to connect the electrodes back to the implantable pulse generation devices have limited the number and utility of electrodes.

For example, each electrode requires an electrical conductor or wire to extend from the electrode through its associated lead and back to the implanted device. The large number of such conductors is limited by the amount of space available in a lead. Further, each conductor requires a hermetically sealed connection with the implanted device. This places a huge burden on feed-through systems which can accommodate only a limited number required contacts and in effect, limits the number of electrodes to the constraints imposed by the connector.

Standard technology includes conductor wire for lead catheters, toroidal spring connectors between the lead and the implantable pulse generator devices, and a hermetic feed-through constructed from metal pins and ceramic insulators. Alternative designs could include improvement to the technology for these methods of connecting the lead to the implantable pulse generator device, by providing higher density connections through miniaturization. Higher density electrodes may now be designed through the use of thin film deposition technology to establish higher density electrodes as well as the high density interconnect conductors.

Still further, the required higher density of conductors required for the increased number of electrodes results in smaller diameter conductors. The smaller diameter conductors present higher impedance conduction paths between the electrodes and the implantable devices. This results in higher required power output from the implantable electronic devices to deliver the desired effective stimulation therapy. The required higher power output also either decreases battery life of the implantable devices or requires larger batteries to be employed. The smaller diameter conductor wire would also exhibit reduced strength and flex life in locations where this results in reduced reliability of the cable lead. Such stresses at the lead/stimulator connections cause an unacceptably high rate of device failure.

As may be seen from the foregoing, there is a need in the art for a different approach in providing therapy within a body where electric therapy is delivered from an implantable pulse generator device to a high density of electrodes. It would be desirable if such an approach would avoid high impedance conduction paths, minimize electrode dislodgement, prevent interconnection issues and increase the safety to and convenience of the patient. The present invention addresses these and other issues.

US-A-2010/0114278, US-A-2014/180370, and US-A-2012/215287 disclose that circuitry for generating stimulation pulses is located outside of a stimulation lead.

### SUMMARY

The present invention provides a neurostimulator system as set out in claim 1.

According to an embodiment, a neurostimulator system includes a pulse generator module and a power source and control module. The pulse generator module includes an electrical stimulation lead and electrodes and is configured to be implanted within a body of a subject, to provide a therapy to the subject, and to receive power wirelessly from a source remote from the pulse generator module. And the power source and control module is configured to be located external to the body of the subject, to cause the pulse generator module to effect the therapy, and to provide power wirelessly to the pulse generator module.

According to one embodiment, a stimulation lead for connecting a pulse generator having a plurality of outputs to electrodes of an electrode array includes a flexible body and the electrode array. The electrode array is distal to the flexible body and the flexible body has a proximal portion and an interface portion. The lead further includes a plurality of conductors extending through the proximal portion to the interface portion, a connector arranged to connect a proximal end of each one of the conductors to a respective given one of the outputs, and a selection circuit within the interface portion. The selection circuit has a plurality of inputs. Each input of the selection circuit is connected to a distal end of a respective given one of the conductors. The selection circuit further has a plurality of outputs. Each output of the selection circuit is coupled to a respective one of the electrodes of the electrode array. The plurality of outputs of the selection circuit are greater in number than the plurality of outputs of the pulse generator. A power supply is configured to receive input power wirelessly from an external source, to generate from the input power at least one power supply signal, and to provide the at least one power supply signal to at least the pulse generator and the selection circuit.

The electrode array may include a flexible substrate and the electrodes of the electrode array may be distributed and carried on the flexible substrate. The flexible substrate may be configured as a cylinder. The electrode array may include a flexible cylindrical carrier, and the flexible substrate may be wrapped about the flexible cylindrical carrier. The flexible cylindrical carrier may be formed of silicone. The flexible substrate may be substantially planar and have a paddle configuration.

The electrode array may include a backing layer arranged in a corkscrew configuration and the flexible substrate may be carried on the backing layer within the corkscrew configuration.

The selection circuit may include a switching array. The switching array may comprise an integrated circuit.

In another embodiment, a universal stimulation lead module for connecting a pulse generator having a plurality of outputs to electrodes of an electrode array includes a flexible body having a proximal portion and a distal interface portion, a plurality of conductors extending through the proximal portion to the interface portion, a connector arranged to connect a proximal end of each one of the conductors to a respective given one of the outputs, and a selection circuit within the interface portion. The selection circuit has a plurality of inputs, each input of the selection circuit being connected to a distal end of a respective given one of the conductors. The selection circuit further has a plurality of outputs, each output of the selection circuit being arranged to be coupled to a respective one of the electrodes of the electrode array. The plurality of outputs of the selection circuit are greater in number than the plurality of outputs of the pulse generator. A power supply is configured to receive input power wirelessly from an external source, to generate output power from the input power, and to provide the output power to at least the pulse generator and the selection circuit.

The selection circuit may include a switching array. The switching array may comprise an integrated circuit.

In another embodiment, a stimulation lead provides stimulation energy to selected ones of a plurality of electrodes of an electrode array under control of a control device having a plurality of outputs that provide power and control signals. The stimulation lead includes a flexible body and the electrode array. The electrode array is distal to the flexible body. The flexible body has a proximal portion and an interface portion. The lead further includes a plurality of conductors extending through the proximal portion to the interface portion, a connector arranged to connect a proximal end of each one of the conductors to a respective given one of the outputs and a pulse generator within the interface portion. The pulse generator is responsive to the power and control signals of the control device to generate the stimulation energy. The lead further includes a selection circuit also within the interface portion. The selection circuit is coupled to the pulse generator and further has a plurality of outputs. Each output of the selection circuit is coupled to a respective one of the electrodes of the electrode array. The selection circuit is arranged to provide selected ones of the electrodes with the stimulation energy responsive to the control signals from the control device. The plurality of outputs of the selection circuit are greater in number than the plurality of outputs of the control device.

The electrode array may include a flexible substrate and the electrodes of the electrode array may be distributed and carried on the flexible substrate. The flexible substrate may be configured as a cylinder. The electrode array may include a flexible cylindrical carrier, and the flexible substrate may be wrapped about the flexible cylindrical carrier. The flexible cylindrical carrier may be formed of silicone. The flexible substrate may be substantially planar and have a paddle configuration.

The electrode array may include a backing layer arranged in a corkscrew configuration. The flexible substrate may be carried on the backing layer within the corkscrew configuration.

The selection circuit may include a switching array. The switching array may comprise an integrated circuit. The pulse generator comprises an integrated circuit.

In still another embodiment a universal stimulation lead module provides stimulation energy to selected ones of a plurality of electrodes of an electrode array under control of a control device having a plurality of outputs that provide power and control signals. The lead module includes a flexible body having a proximal portion and a distal interface portion, a plurality of conductors extending through the proximal portion to the interface portion, a connector arranged to connect a proximal end of each one of the conductors to a respective given one of the outputs of the control device and a pulse generator within the interface portion. The pulse generator is response to the power and controls signals from the control device to generate the stimulation energy. The selection circuit is also within the interface portion and is coupled to the pulse generator. The selection circuit has a plurality of outputs, each output of the selection circuit being coupled to a respective one of the electrodes of the electrode array. The selection circuit is arranged to provide selected ones of the electrodes with the stimulation energy responsive to the control signals from the control device. The plurality of outputs of the selection circuit are greater in number than the plurality of outputs of the control device.

The selection circuit may include a switching array. The switching array may comprise an integrated circuit. The pulse generator comprises an integrated circuit.

Another embodiment of the implanted neurostimulator is a system which provides power, such as continuous power, from an externally worn device. The power is electromagnetically transferred inductively through coils to provide continuous power to the implanted device. This may replace the need for a rechargeable implanted power source, or may provide another power-source option. The implanted pulse generator is still integrated with the lead for more efficient energy delivery to the electrodes. Wireless communications may be implemented with either near field electromagnetic energy or with RF far field power such as MICS (Medical Implantable Communications Service) methods.

### BRIEF DESCRIPTION OF THE DRAWINGS

The features of the present invention which are believed to be novel are set forth with particularity in the appended claims. The invention, together with further features and advantages thereof, may best be understood by making reference to the following description taken in conjunction with the accompanying drawings, in the several figures of which like reference numerals identify identical elements, and wherein:
**FIG. 1** is a top view of a stimulation lead according to one embodiment of the invention;
**FIG. 2** is a top view of another stimulation lead according to another embodiment of the invention;
**FIG. 3** is a top view of a further stimulation lead according to a further embodiment of the invention;
**FIG. 4** is a perspective view of the electrode array of the stimulation lead of **FIG. 3** according to an embodiment of the invention;
**FIG. 5** is a top view of a universal stimulation lead module according to an embodiment of the invention;
**FIG. 6** is a sectional view of the interface portion of the universal stimulation lead module of **FIG. 5**;
**FIG. 7** is a circuit diagram of an alternative lead embodiment wherein the pulse generator is located on the stimulation lead according to another embodiment of the invention;
**FIG. 8** is a top view of a stimulation lead wherein the pulse generator is located on the stimulation lead according to a further embodiment of the invention;
**FIG. 9** is a sectional view, to an enlarged scale, of the electronics module of the stimulation lead of **FIG. 8**;
**FIG. 10** is a top view, to an enlarged scale, of the electronics module of the stimulation lead of **FIG. 8**; and
**FIG. 11** is a top view of another stimulation lead wherein the pulse generator is located on the stimulation lead according to a further embodiment of the invention;
**FIG. 12** is a circuit diagram of another embodiment wherein the controller and power source are external to the human body and the pulse generator is located on the stimulation lead according to another embodiment of the invention.

### DETAILED DESCRIPTION

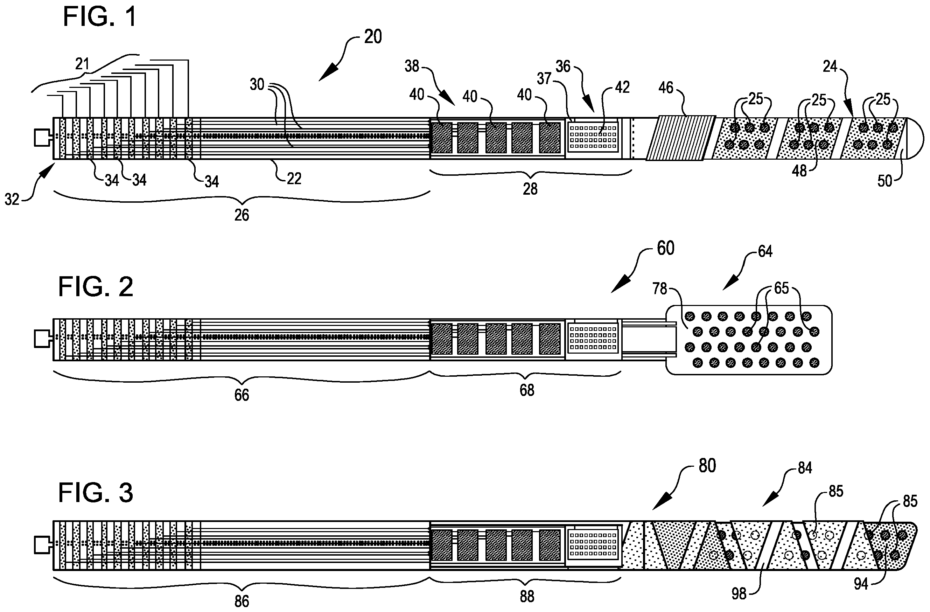

Referring now to **FIG. 1**, it is a top view of a stimulation lead according to one embodiment of the invention. The stimulation lead **20** of **FIG. 1** is arranged for connecting a pulse generator having a plurality of outputs **21** to electrodes **25** of an electrode array **24**. The stimulation lead **20** generally includes a flexible body **22** and the electrode array **24**. The electrode array **24**, which includes a plurality of electrodes **25**, is distal to the flexible body **22**. The flexible body **22** has a proximal portion **26** and an interface portion **28**. The lead **20** further includes a plurality of conductors **30** extending through the proximal portion **26** to the interface portion **28**. The lead **20** still further includes a connector **32** having contacts **34** associated with each of the pulse generator outputs **21**. The contacts **34** are arranged to connect a proximal end of each one of the conductors **30** to a respective given one of the pulse generator outputs **21**.

The lead **20** further includes a selection circuit **36** within the interface portion **28**. The selection circuit **36** preferably includes an integrated switching array **37** of the type known in the art. The selection circuit **36** has a plurality of inputs **38** in the form of connection pads **40**. Each of the connection pads **40** is connected to a distal end of a respective given one of the conductors **30**. The selection circuit **36** further has a plurality of outputs **42**. Each of the outputs **42** of the selection circuit **36** is coupled to a respective one of the electrodes **25** of the electrode array **24** by conductors **46**. As may be noted in **FIG. 1**, the plurality of outputs **42** of the selection circuit are greater in number than the plurality of outputs **21** of the pulse generator.

The electrode array **24**, in this embodiment, takes the form of electrodes **25** deposited on a flexible thin film substrate **48**. The thin film substrate **48** is configured as a cylinder by being wrapped around a flexible rod **50** that may be formed from silicone, for example. The wrapping of the thin film electrode array around the silicone flexible rod **50** provides for improved reliability with lead flex and for improved maneuverability.

**FIG. 2** is a top view of another stimulation lead **60** according to another embodiment of the invention. The lead **60** includes a proximal portion **66** and an interface portion **68** substantially identical to the proximal portion **26** and interface portion **28** of lead **20** of **FIG. 1**. However, here lead **60** includes an electrode array **64** of electrodes **65** deposited on a flexible substrate **78** to form the thin film electrode array **64** in a flat paddle and planar configuration. The electrode array **64** may have a silicone backing **78** to add to directionality, structure, and strength of the thin film electrode array. The paddle electrode configuration could also include a lead introducer catheter, of the type known in the art, to allow the electrode array to be curled up during deployment. Once the catheter is in place, the sheath may be withdrawn allowing the paddle to fold out and be optimally positioned in the desired space.

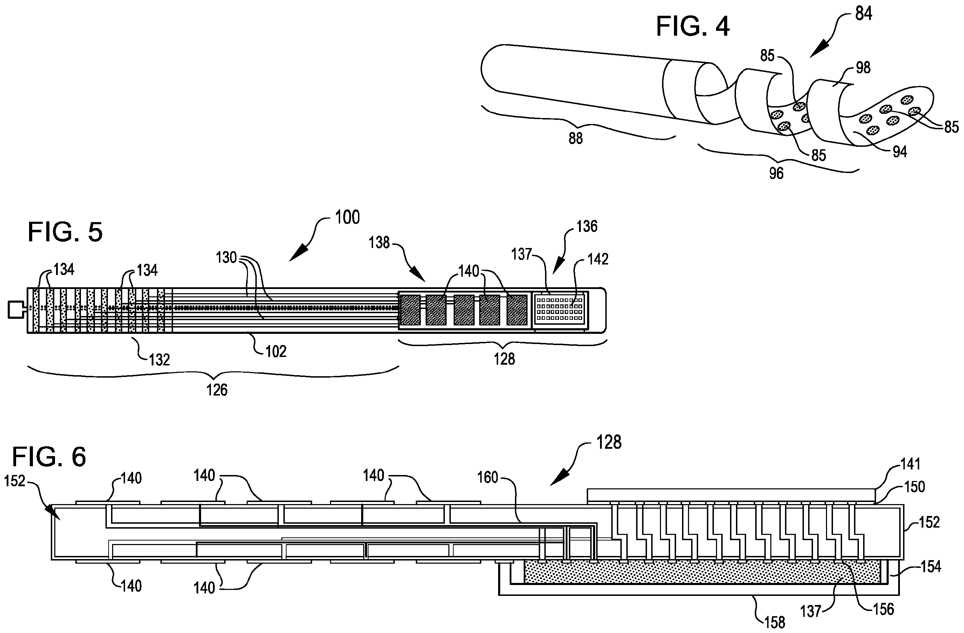

**FIG. 3** is a top view of another stimulation lead **80** according to another embodiment of the invention. The lead **80** includes a proximal portion **86** and an interface portion **88** substantially identical to the proximal portion **26** and interface portion **28** of lead **20** of **FIG. 1**. However, as may be best seen in the perspective view of **FIG. 4**, the lead **80** includes an electrode array **84** of electrodes **85** deposited on a flexible substrate **94** that is carried on the inside of a spiraled silicone backing **98** to form the thin film electrode array **84** in a spiral configuration **96**. This allows the cuff of the electrode array **84** to wrap about an individual nerve with the electrode surface facing the nerve. The silicone backing **98** adds to directionality, structure, and strength of the thin film electrode array **96**.

**FIG. 5** is a top view of a universal stimulation lead module **100** according to an embodiment of the invention. This provides a generic module for a customized electrode array to suit the needs of a specific clinical indication and anatomical site in need of electrical stimulation. More specifically, the universal stimulation lead module **100** of **FIG. 5** may be employed for connecting a pulse generator (not shown) having a plurality of outputs to electrodes of an electrode array. In addition to a customized electrode array, the module **100** may also be used with any one of the electrode arrays disclosed herein. The lead module **100** includes a flexible body **102** having a proximal portion **126** and a distal interface portion **128**. A plurality of conductors **130** extend through the proximal portion **126** to the interface portion **128**. A connector **132** has a plurality of contacts **134**. Each contact **134** is arranged to be connected to a respective given one of the outputs of the pulse generator. The conductors **130**, in turn, connect each respective given one of the contacts **134** to a selection circuit **136** within the interface portion **128**. The selection circuit **136** includes an integrated circuit switching array **137** and has a plurality of inputs **138** in the form of connection pads **140**. Each of the connection pads **140** of the selection circuit **136** is connected to a distal end of a respective given one of the conductors **130**. The selection circuit **136** further has a plurality of outputs **142**. Each output **142** of the selection circuit **136** is arranged to be coupled to a respective one of the electrodes of an electrode array (not shown). The plurality of outputs **142** of the selection circuit **136** are greater in number than the plurality of outputs of the pulse generator and hence the connection contacts **134**.

**Fig. 6** is a sectional view of the interface portion **128** of the universal stimulation lead module **100** of **FIG. 5**. Here it may be seen that the interface portion **128** includes a plurality of layers **152** of a ceramic substrate **156**. The integrated circuit **137** is electrically connected to the bottom side of the interface portion of the ceramic substrate **156**. A titanium or ceramic lid **158** having a gold brazed flange **154** covers the integrated circuit **137** to provide a hermetically sealed enclosure. On top of the interface portion **128** is a thin film array **141** that includes the outputs **142** (**FIG. 5**) of the interface portion **128**. Between the layers **152** of the ceramic are formed conductor paths **160** as, for example, path **160** to interconnect the connection pads **140** to the integrated circuit **137**.

Both integrated circuit **137** and the thin film array **141** may be designed as a grid array for the interconnection process. The ceramic substrate **152** provides a hermetic interconnect between the integrated circuit **137** and external structures to which it will be electrically connected. Staggering the interconnections within the ceramic substrate **156** ensures a hermetic connection for the electrical connections **160**.

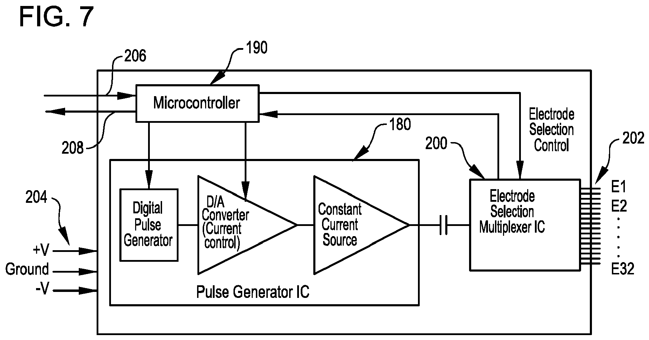

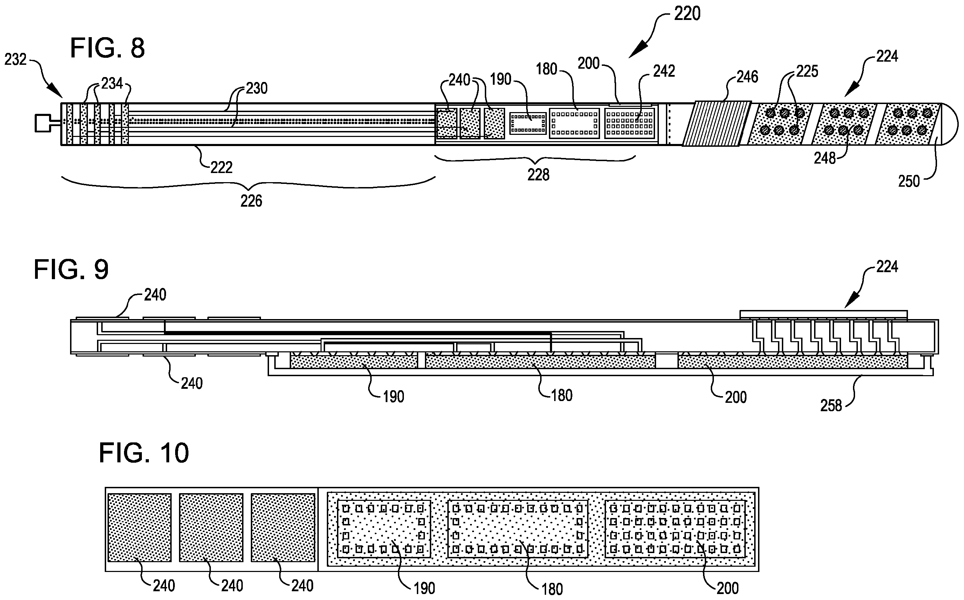

**FIG. 7** is a circuit diagram of an alternative lead embodiment wherein the pulse generator **180** is located on the stimulation lead according to another embodiment of the invention. Along with the pulse generator **180**, in accordance with this embodiment, a microcontroller 190 and an electrode selection integrated circuit 200 are also on the lead 100. A more specific example follows with respect to FIG. 8.

In FIG. 7 it may be seen that the microcontroller 190 is coupled to both the pulse generator 180 and electrode selection circuit 200. The microcontroller 190, responsive to received control signals generated by an implanted power source and control device, determines the parameters of the electrical stimulation provided by the pulse generator 180 and the electrodes to be selected by the electrode selection circuit 200 to which the electrical stimulation is to be applied. In FIG. 7, it may be seen that the electrode selection circuit 200 has for example 32 outputs 202 E1 -E32) while it has only three power inputs 204, one communication input 206 and one communication output 208. Hence with just a few inputs, the lead is able to provide many more electrodes with stimulation. Each of the microcontroller 190, the pulse generator 180, and the selection circuit 200 may be a separate integrated circuit or may be integrated into one circuit.

Referring now to FIG. 8, it is a top view of a stimulation lead 220 wherein the pulse generator 180 is located on the stimulation lead 220 according to a further embodiment of the invention. FIG. 9 is a sectional view, to an enlarged scale, of the electronics module 228 of the stimulation lead 220 of FIG. 8 and FIG. 10 is top view, to an enlarged scale, of the electronics module 228 of the stimulation lead 220 of FIG. 8.

The stimulation lead 220 of FIGS. 8-10 provides stimulation energy to selected ones of a plurality of electrodes 225 of an electrode array 224 under control of a control device (not shown) having a plurality of outputs that provide power and control signals. The stimulation lead 220 includes a flexible body 222 and the electrode array 224. The electrode array 224 is distal to the flexible body 222. The flexible body 222 has a proximal portion 226 and an interface portion 228. The lead 220 further includes a plurality of conductors 230 extending through the proximal portion 226 to the interface portion 228. A connector 242 has a plurality of contacts 234 arranged to connect a proximal end of each one of the conductors 230 to a respective given one of the outputs. The lead 220 further includes the pulse generator 180, the microcontroller 190, and the electrode selection circuit 200 within the interface portion. The pulse generator **180** is responsive to the power and control signals of the control device to generate the stimulation energy. The electrode selection circuit **200** is coupled to the pulse generator **180** and further has a plurality of outputs **242**. Each of the outputs **242** of the selection circuit **200** is coupled to a respective one of the electrodes **225** of the electrode array **224**. The electrode selection circuit **200** is arranged to provide selected ones of the electrodes **225** with the stimulation energy responsive to the control signals from the control device. The plurality of outputs **242** of the selection circuit **200** are greater in number than the plurality of outputs of the control device (and thus the number of contacts **234**).

The lead **220** may be fabricated in the same manner as that described with respect to **FIG. 6**. Also, the proximal portion **226** and interface portion **228** may be fabricated without an electrodes array so that together they may form a universal lead module having a pulse generator **180** therein for use with any desired electrode array. Further, as in the previous embodiments, the electrode array **224** may include a flexible substrate **248** and the electrodes **225** of the electrode array **224** may be distributed and carried on the flexible substrate **248**. The flexible substrate **248** may be configured as a cylinder. The electrode array **224** may include a flexible cylindrical carrier **250,** and the flexible substrate **248** may be wrapped about the flexible cylindrical carrier. The flexible cylindrical carrier **250** may be formed of silicone.

The electrode array **224** may include a backing layer as shown in the embodiment of **FIGS. 3** **and** **4** arranged in a corkscrew configuration. The flexible substrate may be carried on the backing layer **98** within the corkscrew configuration.

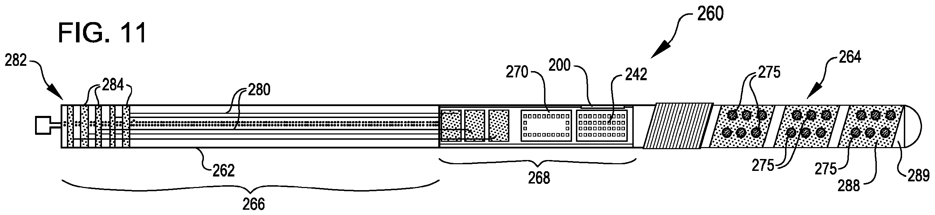

**FIG. 11** is a top view of another stimulation lead **260** wherein the pulse generator **180** is located on the stimulation lead **260** according to a further embodiment of the invention. Here, both the pulse generator **180** and microcontroller **190** are integrated into a common integrated circuit **270**. As in the previous embodiments, the stimulation lead **260** includes a flexible body **262** and an electrode array **264**. The electrode array **264** is distal to the flexible body **262**.

The flexible body **262** has a proximal portion **226** and an interface portion **228,** which portions may be constructed and fabricated as previously described. To that end, the lead further includes a plurality of conductors **280** extending through the proximal portion **266** to the interface portion **268**. A connector **282** has a plurality of contacts **284** arranged to connect a proximal end of each one of the conductors **280** to a respective given one of the outputs of a control device (not shown). The lead **260** further includes the pulse generator **180** and microcontroller **270** and the electrode selection circuit **200** within the interface portion **268**. The pulse generator **180** is responsive to the power and control signals of the control device to generate the stimulation energy. The electrode selection circuit **200** is coupled to the pulse generator **180** and further has a plurality of outputs **242**. Each of the outputs **242** of the electrode selection circuit **200** is coupled to a respective one of the electrodes **275** of the electrode array **264**. The electrode selection circuit **200** is arranged to provide selected ones of the electrodes **275** with the stimulation energy responsive to the control signals from the control device. The plurality of outputs **242** of the electrode selection circuit **200** are greater in number than the plurality of outputs of the control device (and thus the number of contacts **284**).

The lead **260** may be fabricated in the same manner as that described with respect to **FIG. 6**. Also, the proximal portion **266** and interface portion **268** may be fabricated without an electrode array so that together they may form a universal lead module having a pulse generator **180** therein for use with any desired electrode array. Further, as in the previous embodiments, the electrode array **264** may include a flexible substrate **288** and the electrodes **275** of the electrode array **264** may be distributed and carried on the flexible substrate **288**. The flexible substrate **288** may be configured as a cylinder. The electrode array may include a flexible cylindrical carrier **289,** and the flexible substrate **288** may be wrapped about the flexible cylindrical carrier. The flexible cylindrical carrier **289** may be formed of silicone.

The electrode array **264** may include a backing layer as shown in the embodiment of **FIGS. 3** **and** **4** arranged in a corkscrew configuration. The flexible substrate **288** may be carried on the backing layer **98** within the corkscrew configuration.

**FIG**. **12** is a circuit diagram of another embodiment wherein the controller and power source 350 is located external to the body of a subject, such as a human body, according to another embodiment of the invention.

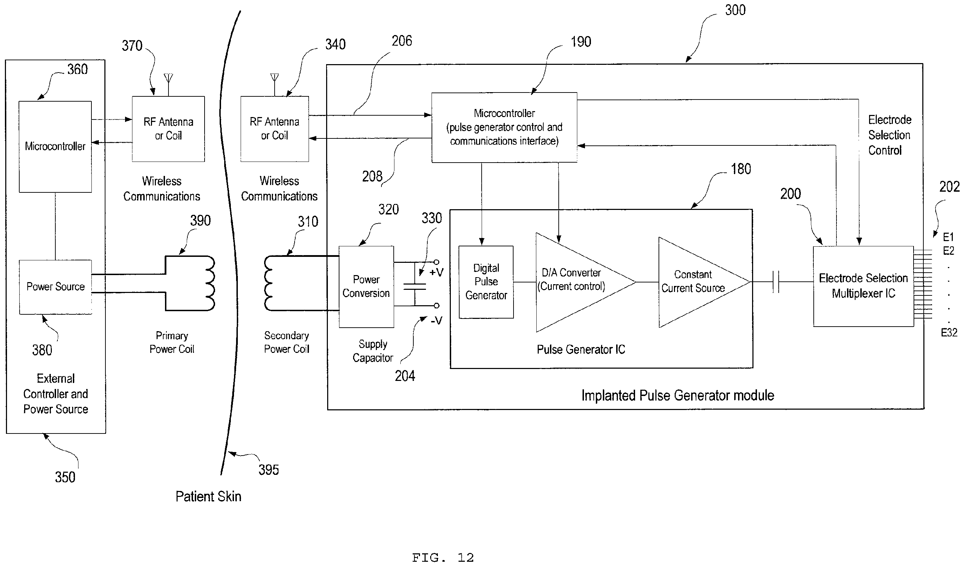

In **FIG. 12** it may be seen that a pulse generator module **300** is implanted in the human body. The microcontroller **190** is coupled to both the pulse generator **180** and electrode selection circuit **200**. The microcontroller **180,** responsive to received control signals generated by an implanted power source and control device, determines the parameters of the electrical stimulation provided by the pulse generator **180** and the electrodes to be selected by the electrode selection circuit **200** to which the electrical stimulation is to be applied. In **FIG. 12****,** it may be seen that the electrode selection circuit **200** has for example **32** outputs **202** (E1-E32) while it has only as few as two power inputs **204** one control input **206** and one control output **208** that extend to outside of the lead (not shown in FIG. 12), into which the electrodes 202 extend. Hence with just a few inputs, the lead is able to provide many more electrodes with stimulation. Each of the microcontroller **190,** the pulse generator **180,** and the selection circuit **200** may be a separate integrated circuit or may be integrated into one circuit. The implanted pulse generator module **300** receives power transcutaneously via inductive coupling from an external controller and power source **350** through electromagnetic coupling between a primary coil **390** and a secondary coil **310.** The power is converted with a power conversion circuit **320** to the required voltages to power the circuits of the implanted pulse generator module **300**. The secondary coil **310** is implanted beneath the subject's skin **395**. The primary coil **390** transmits power to the secondary coil **310** from the external power source **380** which consists of power conversion circuits, an external battery, and which may be configured to receive power (*e.g*., to charge the battery or to provide power to the power source 380 directly) from an adapter such as an AC adapter. The microcontroller **360** determines the amount of power to be generated by the power source **380** as provided to the primary coil **390**. The secondary coil **310** provides the received power to a power conversion circuit **320** which converts the power to DC voltages required to power the pulse generator IC **180,** microcontroller **190,** the electrode selection multiplexer circuit **200,** and any other circuits within the module 300. A supply output capacitor **330** may be large enough to store and provide reserve power for situations of temporary unexpected power interruptions or discontinuities from the external wireless power transfer; alternatively, the module 300 may include a battery that is rechargeable by the power converter 320 and that is configured to provide reserve power for situations of unexpected power interruptions, or that is configured to provide reserve power during a power interruption in response to the capacitor discharging to a level at which it can no longer provide sufficient power to the pulse generator module 300. The microcontroller **360** also transmits and receives wireless communication to and from the implanted pulse generator module **300**. The wireless communication may be implemented as an RF far field method (*e.g*., Medical Information Communications Service, MICS protocol) or with near field electromagnetic data transfer. A two way wireless communications occurs between the external RF antenna or coil **370**, and the implanted RF antenna or coil **340**.

While particular embodiments have been shown and described, modifications may be made, and it is therefore intended to cover all such changes and modifications which fall within the true scope of the invention as defined by the claims.

## Claims

1. A neurostimulator system, comprising:
a pulse generator module (300) including an electrical stimulation lead (220), a pulse-generator circuit (180) and electrodes (202), and configured to be implanted within a body of a subject, to provide a therapy to the subject, and to receive power wirelessly from a source (380) remote from the pulse generator module (300); and
a power source and control module (350) configured to be located external to the body of the subject, to cause the pulse generator module (300) to effect the therapy, and to provide power wirelessly to the pulse generator module (300),
**characterized in that**
the pulse-generator circuit (180) is disposed within the electrical stimulation lead (220).

2. The system of claim 1, wherein the power source and control module (350) is configured to provide to the pulse generator module (300), wirelessly, programming of operational parameters of the pulse generator module (300).

3. The system of claim 1, wherein the pulse generator module (300) is configured to provide, wirelessly, to the power source and control module (350) feedback regarding power usage of the pulse generator module (300).

4. The system of claim 1, wherein the pulse generator module (300) includes a power supply capacitor (330) which is configured to provide reserve power to the pulse generator module (300) during temporary interruptions of wireless power transfer from the power source and control module (350) to the pulse generator module (300).

5. The system of claim 1, wherein the pulse generator module (300) includes a rechargeable battery which is configured to provide back up power that allows the pulse generator module (300) to continue delivery of the therapy during interruptions of wireless power transfer from the power source and control module (350) to the pulse generator module (300).

## Patentansprüche

1. Neurostimulatorsystem, das aufweist:
ein Impulsgeneratormodul (300), das eine elektrische Stimulationssonde (220), eine Impulsgeneratorschaltung (180) und Elektroden (202) aufweist und konfiguriert ist, in einen Körper eines Subjekts implantiert zu werden, um eine Therapie für das Subjekt bereitzustellen, und drahtlos Energie von einer von dem Impulsgeneratormodul (300) entfernten Quelle (380) zu empfangen; und
ein Energiequellen- und Steuermodul (350), das konfiguriert ist, außerhalb des Körpers des Subjekts angeordnet zu sein, um das Impulsgeneratormodul (300) zu veranlassen, die Therapie zu bewirken, und drahtlos Energie an das Impulsgeneratormodul (300) zu liefern,
**dadurch gekennzeichnet, dass**
die Impulsgeneratorschaltung (180) in der elektrischen Stimulationssonde (220) angeordnet ist.

2. System nach Anspruch 1, wobei das Energiequellen- und Steuermodul (350) konfiguriert ist, dem Impulsgeneratormodul (300) zu ermöglichen, dass Betriebsparameter des Impulsgeneratormoduls (300) drahtlos programmiert werden.

3. System nach Anspruch 1, wobei das Impulsgeneratormodul (300) konfiguriert ist, drahtlos an das Energiequellen- und Steuermodul (350) eine Rückmeldung bezüglich des Stromverbrauchs des Impulsgeneratormoduls (300) zu liefern.

4. System nach Anspruch 1, wobei das Impulsgeneratormodul (300) einen Energieversorgungskondensator (330) enthält, der konfiguriert ist, dem Impulsgeneratormodul (300) während vorübergehender Unterbrechungen der drahtlosen Energieübertragung von dem Energiequellen- und Steuermodul (350) an das Impulsgeneratormodul (300) Reserveenergie zu liefern.

5. System nach Anspruch 1, wobei das Impulsgeneratormodul (300) eine wiederaufladbare Batterie aufweist, die dazu konfiguriert ist, Unterstützungsenergie bereitzustellen, die es dem Impulsgeneratormodul (300) ermöglicht, die Durchführung der Therapie während Unterbrechungen der drahtlosen Energieübertragung von dem Energiequellen- und Steuermodul (350) an das Impulsgeneratormodul (300) fortzusetzen.

## Revendications

1. Système neuro-stimulateur, comprenant :
un module générateur d'impulsions (300) comportant une sonde de stimulation électrique (220), un circuit générateur d'impulsions (180) et des électrodes (202) et configuré pour être implanté dans un corps d'un sujet, pour fournir une thérapie au sujet, et pour recevoir de l'énergie sans fil à partir d'une source (380) éloignée du module générateur d'impulsions (300) ; et
un module de commande et source d'énergie (350) configuré pour être situé à l'extérieur du corps du sujet, pour amener le module générateur d'impulsions (300) à effectuer la thérapie, et pour fournir de l'énergie sans fil au module générateur d'impulsions (300),
**caractérisé en ce que**
le circuit générateur d'impulsions (180) est disposé dans la sonde de stimulation électrique (220).

2. Système selon la revendication 1, dans lequel le module de commande et source d'alimentation (350) est configuré pour fournir, sans fil, au module générateur d'impulsions (300) une programmation de paramètres opérationnels du module générateur d'impulsions (300).

3. Système selon la revendication 1, dans lequel le module générateur d'impulsions (300) est configuré pour fournir, sans fil, au module de commande et source d'énergie (350) une rétroaction concernant l'utilisation d'énergie du module générateur d'impulsions (300).

4. Système selon la revendication 1, dans lequel le module générateur d'impulsions (300) comporte un condensateur d'alimentation (330) qui est configuré pour fournir une alimentation de réserve au module générateur d'impulsions (300) pendant des interruptions temporaires de transfert d'énergie sans fil depuis le module de commande et source d'énergie (350) au module générateur d'impulsions (300).

5. Système selon la revendication 1, dans lequel le module générateur d'impulsions (300) comporte une batterie rechargeable qui est configurée pour fournir une puissance de secours qui permet au module générateur d'impulsions (300) de continuer l'administration de la thérapie pendant des interruptions de transfert d'énergie sans fil depuis le module de commande et source d'énergie (350) au module générateur d'impulsions (300).
